# EUROPEAN PATENT APPLICATION

(11) **EP 1 260 238 A2**
(43) Date of publication of application: **27.11.2002**
(21) Application number: 02010296.8
(22) Date of filing: 21.05.2002
(51) Int. Cl.: A61M 1/36

(54) **Blood collection device**

(30) Priority: 22.05.2001 IT BO20010320
(71) Applicant: Balbo, Enrico, 41100 Modena (IT)
(72) Inventor: Balbo, Enrico, 41100 Modena (IT)
(74) Representative: Modiano, Guido, Dr.-Ing.

(57) **Abstract**

A blood collection device comprises: a first lower portion (11), which is adapted to contain blood and air; a second upper portion (12), which is adapted to contain air; an elastic diaphragm (17), which is interposed between the lower portion (11) and the upper portion (12) and can be deformed due to the difference between the pressure of the air that is present in the lower portion (11) and the pressure of the air in the upper portion (12); and a blood transfer connector (15).

## Description

The present invention relates to a blood collection device to be used in circuits for hemodialysis and for extracorporeal blood circulation.

Blood collection devices, known as drip chambers, venous chambers or arterial chambers, are known which are used in particular in hemodialysis circuits and are made of rigid and/or flexible transparent plastics, with predominantly cylindrical shapes.

Blood collection devices which are crossed by the blood sent to and/or arriving from any blood processing device perform the following functions:
-- They act as blood accumulation reservoirs; by means of particular access tubes, it is possible to regulate the level of the blood inside them according to the flow-rate and pressure of the stream of blood that flows through them.
-- They are used to draw samples of blood to be analyzed and/or to infuse anticoagulants and drugs to the patient.
-- They act as chambers for separating the air from the blood stream that passes through them.
-- They allow continuous measurement of the pressure of the blood contained in them; in current clinical practice, the pressure and its variations are measured by means of pressure gauges located on the panels of the units that regulate and monitor extracorporeal circulation processes. The compressed air that arrives from the collection device and is propelled by the variations in the level and pressure of the blood rises along the tube that connects the collection device and the pressure gauge of the apparatus, actuating the internal transducer of the pressure gauge.

A drawback of this type of pressure measurement is that the blood may reach and flood the internal body of the pressure transducer of the pressure gauge on the extracorporeal circulation monitoring apparatus.

This entails laborious cleaning and disinfection operations or even the replacement of the measurement unit or transducer of the pressure gauge.

In order to obviate this drawback, so-called blood catchers or transducer protectors have been marketed; these devices are provided with a porous and liquid-repellent diaphragm that prevents accidental contact between the blood and the inside of the pressure gauge.

The blood barrier effect performed by blood catchers with a filtering liquid-repellent diaphragm, however, is not sufficient to prevent viral contaminations in the presence of volume displacements of air that has come into contact with blood of patients affected by particular disorders.

As an alternative, devices have been developed which have a nonfiltering deformable diaphragm; under pressure, the diaphragms undergo deformation and, by displacing a certain volume of air, transmit the pressure variation to the pressure gauge of the apparatus.

In both of the above described cases currently in use, it is necessary to manufacture and use a separate device to be added to the conventional blood collection devices.

The aim of the present invention is to eliminate the drawbacks of conventional devices, by providing a blood collection device that allows to eliminate conventional blood catchers, simplify hemodialysis lines, and ensure a viral barrier.

Within this aim, an object of the present invention is to devise a structure that is simple, relatively easy to provide in practice, safe in use, effective in operation, and relatively low in cost.

This aim and this and other objects that will become better apparent hereinafter are achieved by the present blood collection device, characterized in that it comprises: a first lower portion, which is adapted to contain blood and air; a second upper portion, which is adapted to contain air; an elastic diaphragm, which is interposed between said lower portion and said upper portion and can be deformed due to the difference between the pressure of the air that is present in said lower portion and the pressure of the air in said upper portion; and blood transfer means.

Further characteristics and advantages of the present invention will become better apparent from the detailed description of a preferred but not exclusive embodiment of a blood collection device, illustrated only by way of non-limitative example in the accompanying drawings, wherein:
Figure 1 is a sectional view of the blood collection device according to the invention;
Figure 2 is a partially sectional axonometric view of details of the device of Figure 1;
Figure 3 is a view of the details of Figure 2, taken along the direction A.

With reference to the figures, the reference numeral 10 generally designates a blood collection device according to the present invention.

The device 10 can be ideally divided into a lower element 11 and an upper element 12, which are connected one another so as to form a space 13 that is filled with air in the upper region and filled with air and blood in the lower region.

The lower element 11 comprises a tubular portion 11a, which is connected to a substantially cylindrical portion 11b by a funnel 11c.

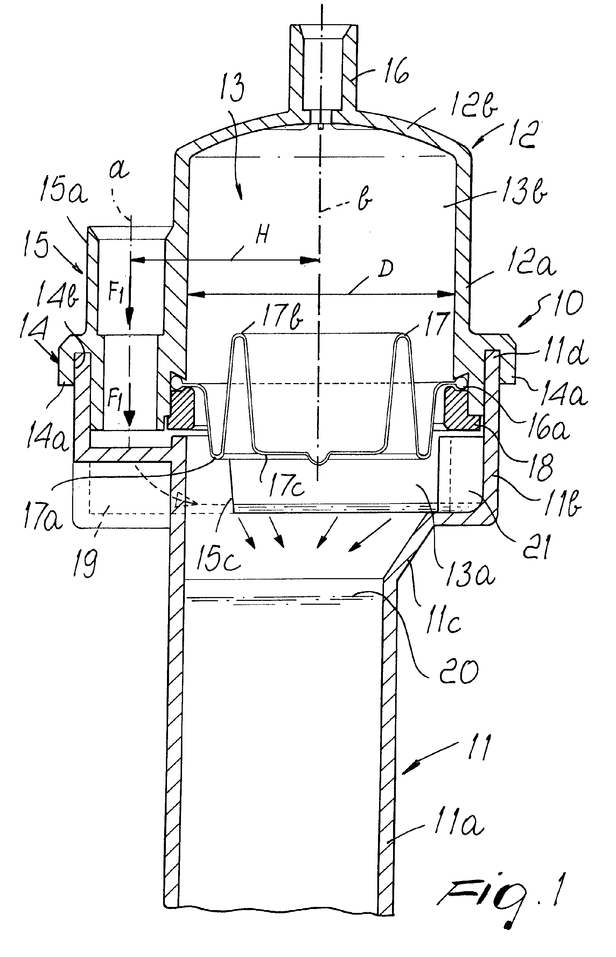

In the embodiment of Figure 1, the portions 11a and 11b and the funnel 11c are made of a transparent plastic material and are obtained monolithically.

The upper element 12 has a substantially cylindrical portion 12a, which is provided with a dome 12b and with means 14 for connection to the lower element 11.

Blood transfer means 15 are provided and distributed between the lower element 11 and the upper element 12.

The dome 12b has a connection port 16, which is connected, by means of a tube (not shown), to a monitoring pressure gauge (not shown) of an apparatus (not shown) that monitors the process of extracorporeal blood circulation.

An elastic diaphragm 17 is inserted between the lower element 11 and the upper element 12 and is shaped as shown by way of non-limitative example, its circular outer rim 16a being locked onto the upper element 12 by a locking ring 18.

Without abandoning the scope of the invention, the elastic diaphragm 17 might be thermally bonded onto the upper element 12 or glued or coupled by means of other known technologies and therefore there might be no need for the locking ring 18.

The elastic diaphragm 17 divides the space 13 into a lower half-space 13a and an upper half-space 13b.

As shown in Figure 1, the plane surface diametral dimensions of the elastic diaphragm 17 are such that they are practically equal to the diameter D of the transverse sectional dimensions of the upper element 12.

The elastic diaphragm 17, made of elastic and impermeable material, is given an accordion-like shape by providing thereon at least two folds 17a and 17b and a portion 17c that is substantially flat and circular.

In the embodiment shown in Figure 1, the means 14 for connecting the upper element 12 to the lower element 11 comprise a lip 14a, which forms an annular interspace 14b in which the upper rim 11d of the portion 11b of the lower element 11 enters and is fixed.

The transfer means 15 comprise a transfer connector or duct 15a, which is substantially tubular and is coupled to the cylindrical portion 12a of the upper element 12.

The connector 15a can be connected, by means of a flexible tube (not shown), together with the remaining part of an extracorporeal blood circulation line, to the device 10.

In the illustrated embodiment, the connector or duct 15a is obtained monolithically with the upper element 12 of the device.

Furthermore, the longitudinal axis (a) of the connector 15a is offset by a distance (H) with respect to a longitudinal axis (b) of the upper element 12.

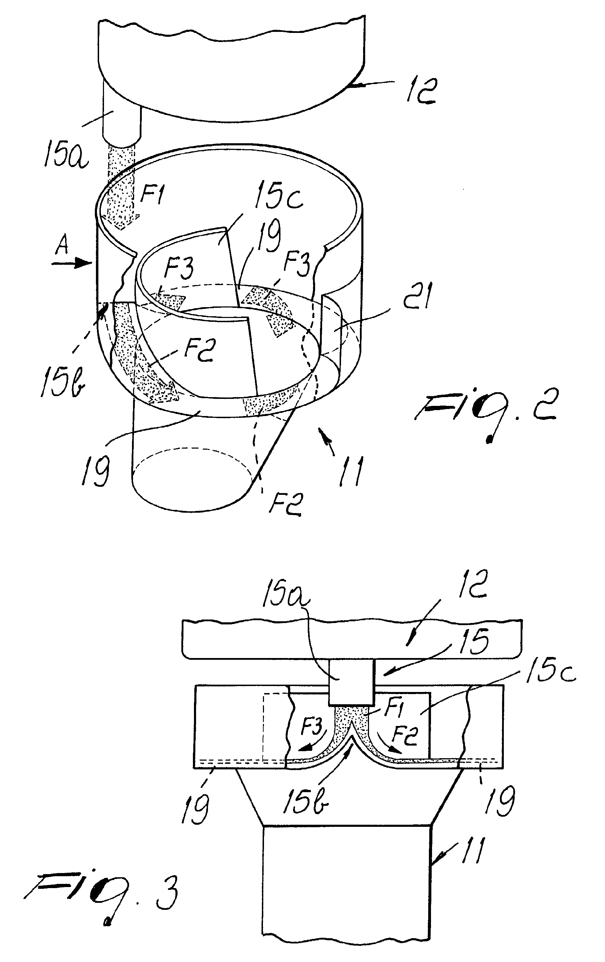

The blood transfer means 15 (see Figure 3) comprise a cusp-shaped diverging separator partition 15b, which by being arranged right in front of the outlet of a stream F1 of blood from the connector 15a, divides such stream F1 along two semicircular portions into two streams F2 and F3.

On either side of the separator partition 15b a respective circular ledge 19 is provided as a semicircular portion which is horizontal in the drawings but needs not to be horizontal, and is rigidly connected to the funnel 11c that is directed downward.

Both the ledges 19 and the funnel 11c are considered to belong to the transfer means 15.

To prevent the blood that flows out of the connector 15a from flowing immediately into the lower element 11, a wall 15c is provided, which has a semicircular plan shape.

In order to avoid interrupting the laminar flow of the blood in the point where the two opposite streams merge, a cusp-shaped converging partition 21 is provided, which is arranged on the opposite side with respect to the separator partition 15b with respect to the wall 15c.

As shown in Figure 2, the connector 15a is formed monolithically with the upper element 12, while the separator partition 15b, the semicircular wall 15c, the converging partition 21 and the brackets or ledges 19 are obtained monolithically with the lower element 11.

The diverging and converging partitions 15b and 21 are shaped according to the principles of fluid dynamics, so as to minimize formation of vortices in the transition from the stream F1 to the streams F2, F3 and in their subsequent remerging, the blood stream being, substantially, under laminar fluid-dynamic conditions.

The generation of turbulences in the streams F1, F2, F3 would entail the inclusion of air particles in the blood, with the risks of embolism that this can cause in the patient being treated.

The presence of the circular ledges 19, in addition to minimizing the turbulence of the blood in transit, facilitates the release of any microbubbles of air that might be included in the blood upstream in the extracorporeal circuit. After passing through the funnel 11c, the blood is arranged by gravity in the lower element 11 and has a free surface 20.

The lower half-space 13a is formed by the space enclosed between the lower surface of the diaphragm 17, the inner wall of the funnel 11c, and the inner wall of the cylindrical portion 11b of the element 11.

The upper half-space 13b is instead formed by the space enclosed between the upper surface of the diaphragm 17, the inner surface of the dome 12b, and the inner surface of the cylindrical portion 12a.

In another embodiment, easily inferable on the basis of the above description, the opposite occurs for a device 10 used in a so-called venous circuit, in which blood is transferred from above. The embodiment in which the blood is transferred from below is particularly advisable for devices 10 to be used in so-called arterial circuits.

At the cylindrical end 11a two ducts are provided, a first one for introducing the blood into the device 10 and a second one for its evacuation therefrom; in this embodiment it is possible to eliminate the ledge 19, the cusp-like protrusions 15b and 21, and the transfer duct 15.

The operation of the blood collection device according to the invention is as follows.

The blood, which enters according to the stream F1, flows over the surface of the ledges 19 and over the surface of the funnel 11c, flows over the inner walls of the cylindrical portion 11a, and collects inside it.

Here, increases in pressure downstream of the lower element 11 are matched by rises of the free surface 20 of the blood inside said lower element 11. These variations of the free surface 20 displace the air contained in the lower half-space 13a, causing the deformation of the diaphragm 17.

The upward deformation of the diaphragm 17, thanks to the reversible flattening of the folds 17a and 17b, is converted into a reduction of the half-space 13b. In turn, this reduction in the volume of the half-space 13b leads to an increase in pressure inside said half-space 13b.

The signal proportional to the pressure increase is transmitted to the pressure gauge of the monitoring machine (not shown) by way of an adapted tube (not shown), which is connected to the port 16.

The opposite phenomenon occurs when the level of the blood inside the lower element 11 decreases.

In this case, a downward deformation of the diaphragm 17 and an increase in the volume of the half-space 13b occur, with a consequent reduction of the pressure signal.

The diaphragm 17 is deformable, elastic and impermeable, is made of biocompatible material and is designed to transmit positive and negative pressure variation signals thanks to its particular accordion-like shape.

With this solution, regardless of the variation of the free surface 20 of the blood, one eliminates the drawback that can instead occur when using commercial blood-catchers that are external to the collection devices, i.e., that the blood, by rising along the pressure measurement line, makes contact with the surface of the liquid-repellent membrane of the blood catcher, blocking it and preventing correct transmission of the overpressure signal.

Paramedic staff in this case must intervene and replace the blocked blood catcher by cutting the connection tube and inserting a new sterile blood catcher. These operations entail the risk of compromising the sterility of the end portion of the connection to the pressure gauge of the apparatus.

With the present invention the blood cannot rise along the pressure signal line thanks to the physical interposition of the diaphragm 17, and accordingly there is no need to protect the pressure gauge of the extracorporeal circulation control apparatus.

Furthermore, the diaphragm 17 is an effective barrier against virus propagation.

The application of the concept of the separator diaphragm to the trays that are already widely commercially available is very important for the safety of the handling of extracorporeal circulation treatments, since it solves in a simple manner the problem of cross-contamination among patients subjected to hemodialysis or other therapy requiring extracorporeal circulation.

It is known that in extracorporeal blood circulation lines the presence of air in the blood, in the form of microbubbles, can be caused by the drifting of air bubbles trapped in the circuit during its filling, by the release of gas dissolved in the plasma due to temperature variations, by cavitation of the blood in pumping units, and by inflows of air wherever negative pressures occur in the circuit.

It is also known that the presence of air in the blood that returns to the patient is a source of risk, since it can cause gaseous embolism.

In principle, therefore, prolonged transit on a thin sheet of blood, as occurs in the semicircular ledges 19, provides microbubbles with a better opportunity to coalesce and dissolve at the surface of the sheet, since said blood sheet has a flow-rate that is half the initial one and a consequently lower thickness.

Auxiliary factors related to the particular design of the ledge 19 for the descent of the blood into the lower element 11, such as centrifugal separation and so-called skimming, can further contribute to the elimination of microbubbles.

Due to coalescence, the air is collected in increasingly large bubbles and accumulates in the half-space 13a.

In this manner, an effective system for transferring and collecting the blood into the lower element 11 has been provided which, in addition to allowing the passage of the blood without interfering with the elastic diaphragm, offers the advantages of not subjecting blood to traumatic treatment and of effectively extracting the air dissolved therein.

In practice it has been found that the described invention achieves the intended aim and objects.

The invention thus conceived is susceptible of numerous modifications and variations, all of which are within the scope of the appended claims.

All the details may further be replaced with other technically equivalent ones.

In practice, the materials used, as well as the shapes and the dimensions, may be any according to requirements without thereby abandoning the scope of the protection of the appended claims.

The disclosures in Italian Patent Application No. BO2001A000320 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A blood collection device, **characterized in that** it comprises: a first lower portion (11), which is adapted to contain blood and air; a second upper portion (12), which is adapted to contain air; an elastic diaphragm (17), which is interposed between said lower portion (11) and said upper portion (12) and can be deformed due to the difference between the pressure of the air that is present in said lower portion (11) and the pressure of the air in said upper portion (12); and blood transfer means (15).

2. The device according to claim 1, **characterized in that** said transfer means (15) comprise a connector (15a) for transferring the blood stream (F1) into said device (10), whose longitudinal axis (a) is offset by a preset distance (H) with respect to the longitudinal axis (b) of said upper portion (12), and two semicircular portions (19), so that the incoming stream (F1) of blood has substantially laminar fluid-dynamics conditions.

3. The device according to one or more of the preceding claims, **characterized in that** said transfer means (15) comprise a diverging partition (15b) for separating a stream of blood (F1) into two streams (F2, F3), a semicircular wall (15c), two symmetrical ledges (19) and a converging partition (21).

4. The device according to one or more of the preceding claims, **characterized in that** said elastic diaphragm (17) forms a lower half-space (13a), which is adapted to contain blood and air, and an upper half-space (13b), which is adapted to contain air.

5. The device according to one or more of the preceding claims, **characterized in that** said elastic diaphragm (17) is constituted by elastic and impermeable material which is folded in an accordion-like fashion and has at least two folds (17a, 17b) and a substantially flat circular portion (17c).

6. The device according to one or more of the preceding claims, **characterized in that** the surface diametral dimensions of said diaphragm (17) are substantially equal to the cross section diameter of said second upper portion (12).

7. The device according to one or more of the preceding claims, **characterized in that** said transfer duct (15a) is obtained monolithically with said second upper portion (12).

8. The device according to claim 3, wherein said diverging and converging partitions (15b, 21), said semicircular wall (15c) and said ledges (19) are obtained monolithically with said first lower portion (11).

9. The device (10) according to any one of the preceding claims, adapted to be used as a venous tray.

10. The device (10) according to any one of the preceding claims, adapted to be used as an arterial tray.
